# EUROPEAN PATENT SPECIFICATION

(11) **EP 2 146 757 B1**
(45) Date of publication and mention of the grant of the patent: **25.05.2011**
(21) Application number: 08745022.7
(22) Date of filing: 03.04.2008
(51) Int. Cl.: A61L 31/02, A61L 31/08, A61L 31/16

(54) **STENTS WITH DRUG RESERVOIR LAYER AND METHODS OF MAKING AND USING THE SAME**
STENTS MIT ARZNEI-RESERVOIRSCHICHT UND VERFAHREN ZU IHRER HERSTELLUNG UND VERWENDUNG
ENDOPROTHÈSES AVEC COUCHE RÉSERVOIR DE MÉDICAMENT ET PROCÉDÉS DE FABRICATION ET D'UTILISATION DE CELLES-CI

(30) Priority: 06.04.2007 US 697401
(43) Date of publication of application: 27.01.2010
(73) Proprietor: Boston Scientific Limited, Christ Church (BB)
(72) Inventor: ATANASOSKA, Liliana, Edina, MN 55436 (US); WEBER, Jan, 6228 GJ Maastricht (NL); WARNER, Robert, W,, Woodbury, MN 55126 (US)
(74) Representative: Peterreins, Frank
(86) International application number: PCT/US2008/059280
(87) International publication number: WO 2008/124513

(56) References cited:
- WO-A-2005/082277
- WO-A-2007/133520
- WO-A-2008/024477
- WO-A-2008/033711
- WO-A-2008/106223
- US-A- 5 980 566
- US-A1- 2005 021 127
- US-A1- 2007 036 905
- US-A1- 2007 071 789

## Description

### Technical Field

This disclosure relates to stents and related methods. Specific arrangements also relate to methods and configurations of stents with drug reservoir layers.

### Background

Stents are prosthetic devices typically intraluminally placed by a catheter within a vein, artery, or other tubular body organ for treating conditions such as, occlusions, stenoses, aneurysms, dissection, or weakened, diseased, or abnormally dilated vessel or vessel wall, by expanding the vessel or by reinforcing the vessel wall. Stents can improve angioplasty results by preventing elastic recoil and remodeling of the vessel wall and treating dissections in blood vessel walls caused by balloon angioplasty of coronary arteries.

Stents are typically tubular and expandable from a collapsed state to an expanded state. In a typical operation to implant a stent, the stent is initially configured in the collapsed state, with a cross-sectional size sufficiently small for ease of passage to the intended site. After the stent reaches the intended site, the stent is typically deformed to increase its cross-sectional size to fully engage the stent with the surrounding tissues. The stent thereafter remains in place in the expanded state.

In some cases, stents are loaded with drugs to be released over time to treat various conditions. Drugs are typically dispersed in coating layers on the surfaces of stents.

While conventional stent technology is relatively well developed, technologies related to drug-delivering stents are still being developed.

US 2007/071789 A1 discloses implantable medical devices employing a sol-gel composition coatings that functions as a bioactive material reservoir, and the use of sol-gel composition coatings for improved adhesion of organic and inorganic substrates.

WO 2005/082277 discloses sustained-release drug-delivery devices employing a mesoporous oxide coating that functions as a drug reservoir, and the use of mesoporous oxide coatings for improved adhesion of organic polymers to inorganic substrates.

US 2005/021127 A1 discloses an implantable medical device that comprises an attached porous ceramic component. This ceramic component joins the surface of the medical device to an auxiliary component such as a glass, plastic, ceramic, or metal device.

US-A-5 980 566 discloses a vascular stent including an elongate biocompatible metal member of cylindrical shape and tubular sidewall with a pattern of multiple openings therethrough and open ends. The sidewall has a thin adherent coating of iridium oxide covering substantially its entire exposed surface. The iridium oxide coating has a biodegradable carrier of drugs applied thereto for localized action.

US 2007/036005 A1 discloses spray processes used to form porous coatings on consolidated biocompatible medical devices. The porous substrates and coatings may be used as a reservoir to hold a drug or therapeutic agent for elution in the body.

### Summary of the Disclosure

The invention relates to a method according to claim 1 and a stent according to claim 9. Advantageous embodiments are described in the dependent claims.

The present disclosure relates generally to a method of making drug reservoir surface coating layers on stent bodies. In one arrangement, the method includes forming a drug containing ceramic film using sol-gel methods.

A further aspect of the present disclosure relates to stents with drug reservoir surface coating layers. In one configuration, stents have surface portions that are configured to be conducive to cell growth on the stent. The stents further have other surface portions that are configured to be conducive to storing drugs and releasing the drugs at desired rates.

### Brief Description of the Drawings

Figure 1 is a schematic perspective view of an example stent constructed according to on aspect of the present disclosure.
Figure 2 is a schematic cross-sectional view of a portion of the stent shown in Figure 1.
Figure 3 is a schematic diagram showing the formation of a drug-polymer complex according to one aspect of the present disclosure.

### Detailed Description

### I. Overview

This disclosure relates to making stents having surface layers, with portions of the surface layers configured as drug reservoir layers, and other portions configured to promote endothelial cell growth, thereby enhancing the integration of stents into their surrounding tissues.

Stents are typically implanted intraluminally in tubular body organs, such as blood vessels, to expand or strengthen the portion of the organ where the stent is placed. It is typically desirable for a stent to have surface portions capable of promoting cell growth over the surfaces of the stent. For drug eluting stents, it is also desirable for a stent to have surface portions capable of storing drugs and releasing the drugs at particular rates. Generally, a set of surface characteristics that are best suited for cell growth may not be best suited for a drug reservoir. For example, a surface layer with a highly porous structure is typically well suited for drug reservoir applications. However, such a structure may not be ideal for cell growth.

One solution according to an aspect of the present disclosure is to provide a stent comprising a stent body with ceramic coating layer having portions of two different porosities. Portions of the stent body surface are etched, e.g., by chemical or laser etching to produce a greater surface roughness than other portions of the stent body. In one aspect of the present disclosure, at least 10% of the nominal total stent body surface area is etched to produce a greater surface roughness than at least another 10% of the nominal total stent body surface area. The nominal total stent body surface area is measured at scales sufficiently greater than the sizes of the pores such that surface roughness due to porosity can be ignored. In other aspects of the disclosure, at least 20% or 30% of the nominal total stent body surface area is etched. The difference in roughness between a first, etched portion and a second portion is generally at least sufficient to enable significant clinically different applications for the two portions, respectively. In one aspect of the present disclosure, the difference is such that the a ceramic layer formed on the first portion is a more suitable drug reservoir layer; and a ceramic layer formed on the second portion is a more suitable substrate for cell growth. According to one aspect of the present disclosure, the first, etched portion of the stent body surface has a higher porosity than the second portion by at least about 50%. The porosity here is measured in volume fraction occupied by the pores. In other aspects of the disclosure the first portion has a higher porosity than the second portion by at least 100%, 500% and 1000%, respectively. A sol-gel process is then used to form ceramic coating layers over the stent body surface portions. The ceramic coating layers over the etched portions of the stent body surface possess a relatively higher porosity (e.g., by at least about 50%, 100% or 500%) than the ceramic coating layers over the non-etched portions of the stent body.

In a further aspect of the present disclosure, portions of a stent, e.g., the abluminal (i.e., away from the lumen, or toward vessel wall) surface portions and at least a portion of the lateral surfaces, are configured as reservoirs to store drugs, such as antirestenotics, antibiotics and anti-proliferative drugs, and to locally release the drugs over time at predetermined rates. Other portions of the stent, e.g., the adluminal (i.e., toward the lumen) surface portions, are configured to promote endothelial cell growth on the stent. In one example drug-eluting stent, the adluminal surface of the stent comprises drug reservoir portions having a porous ceramic layer; the abluminal surface of the stent comprises portions with smooth ceramic coating conducive to endothelial cell growth.

### II. Example Configurations

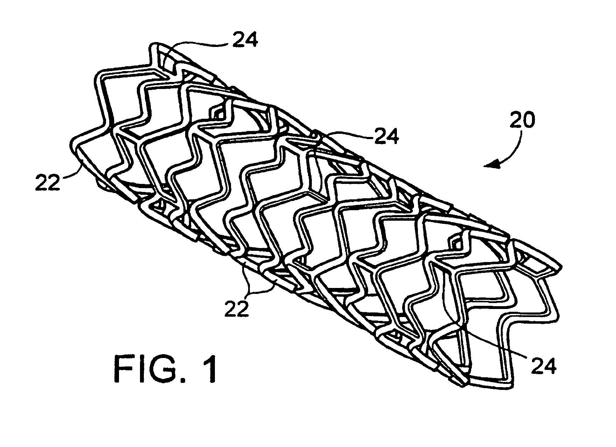

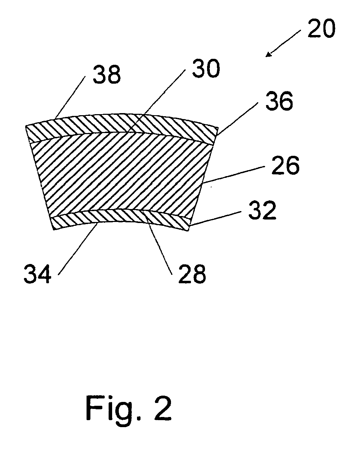

A process for making a stent with drug reservoir coating layers is now described with reference to an example stent 20 in Figures 1 and 2. The stent 20 is shown in an expanded state. The stent 20 has the form of a tubular member defined by a plurality of bands 22 and a plurality of connectors 24 that extend between and connect adjacent bands. During use, bands 22 are expanded from an initial, smaller cross-sectional size to a larger one to contact stent 20 against a wall of a vessel, thereby expanding or strengthening the vessel. A stent can be expanded using a variety of methods. For example, one or more balloons can be used to expand a stent. A self-expanding stent can also be compressed into a collapsed state and held in the collapsed state by a sheath prior to implantation, and unsheathed and permitted to expand at the implantation site. Examples of self-expanding stents include stents made of memory metals, which are flexible and collapsible from a predefined shape at room temperature but regains the predefined shape above certain critical temperature. Connectors 24 provide stent 20 with flexibility and conformability so that the stent can adapt to the contours of the vessel.

As schematically shown in Figure 2, the stent 20 comprises a stent body 26. In accordance with one aspect of the present disclosure, a method for making a stent 20 comprises making surface portions of the stent body 26 rougher than other surface portions and subsequently forming ceramic layers over the stent body surface portions of differing roughness to produce different porosities in the ceramic layers. Surface portions of the stent body 26 can be processed, for example, by chemical etching or laser etching, to produce a greater roughness than other surface portions. Sol-gel processes can be used to form ceramic layers that have greater porosity over the roughened surface portions of the stent body 26 than over other surface portions.

As an example, as schematically shown in Figure 2, the stent body 26 comprises an adluminal surface 28 and abluminal surface 30. The abluminal surface 30 is etched to result in a roughness that is significantly greater than the roughness of the adluminal surface 28. An adluminal ceramic layer 32 is coated on the adluminal surface 28. An abluminal ceramic layer 36 is coated on the abluminal surface 30. Both ceramic layers 32, 36 are formed by a sol-gel process. As a result, the adluminal ceramic layer 32 is a densely packed layer, which is conducive to cell growth; and the abluminal ceramic layer 36 is a porous layer, which can serve as a drug reservoir.

In one aspect of the present disclosure, the porosity of the etched surface portions range from about 15% to about 90%, and the porosity of the unetched surface portions range from about 0% to about 10%. The porosities in this example are expressed in terms of volume fractions occupied by the pores. Various techniques for measuring porosity are known. Examples include helium pycnometry, mercury porosimetry, physical gas adsorption techniques, Rutherford backscattering spectroscopy (RBS) and cyclic voltammetry (CV). Specific examples of porosity measurement techniques include: P. Klobes et al., Porosity and Specific Surface Area Measurement for Solid Materials, NIST Recommended Practice Guide, NIST SP 960-17 (2006); M. Lakatos-Varsanyi et al, Cyclic voltammetry measurements of different single-, bi- and multilayer TiN and single layer CrN coatings on low-carbon-steel substrates, Corrosion Science, Volume 41, Number 8, 1 August 1999, pp. 1585-1598; H.A. Ponte et al, Porosity determination of nickel coatings on copper by anodic voltammetry, Journal of Applied Electrochemistry, Volume 32, Number 6, pp. 641-646 (2002); and V. Torres-Costa et al, RBS characterization ofporous silicon multilayer interference filters, Electrochemical and Solid-State Letters, 7(11), G244-G246 (2004). The above-cited references are incorporated herein by reference.

### A. Surface Treatment of Stent Body

As mentioned above, the textures of the adluminal surface 28 and abluminal surface 30 can be controlled by appropriate surface processing. In one aspect of the present disclosure, the greater roughness of the abluminal surface 30, as compared to that of the adluminal surface 28, is produced by etching the abluminal surface 30. Various metal etching techniques are known and can be used. For example, chemical etching and laser etching methods can be used in the alternative or in combination. For chemically etching stainless steel, for example, a mixture of nitric acid and hydro fluoric acid can be used. Examples of usable laser etching techniques can be found in J. Philip et al., "Laser based etching technique for metallography and ceramography", Materials Science and Engineering A, Vol. 338, 1-2 (2002), pp. 12-23, and A. Kruusing, "Underwater and water-assisted laser processing: Part 2 -- Etching, cutting and rarely used methods", Optics and Lasers in Engineering, 41 (2004), pp. 329-352. Both references above are incorporated herein by reference.

To create surfaces of different roughness with chemical etching, etching resists can be used to cover up the surface portions where etching is not desired. For example, a negative photo-chemical resist can be applied to the stent body 26, and the portions of the resist are irradiated with, for example, ultraviolet light. The irradiated resist is then washed with a developer to remove the unirradiated portions, thereby exposing the corresponding portions of the stent body surface underneath. A photo-chemical resist mask is thus formed, protecting the portions of the stent body 26 covered by the photo-chemical resist from etching. The masked stent body 26 is then subjected to the etching chemicals to etch the portions unprotected by the mask. Suitable photo-chemical etching resists can be obtained from variety of sources, including HTP HiTech Photopolymere AG, Basel, Switzerland.

### B. Formation of Ceramic Surface Coating Layers

According to an aspect of the present disclosure, the ceramic layers 32, 36 are formed by a sol-gel process. The process is based on complex formation between (a) templating agents such as Pluronic F127 nonionic block-copolymer and polyethylene glycol (PEG), (b) hydrolysis/condensation inhibitors such as carboxylic acids, keto-esters and amines, and (c) partially hydrolyzed titanium alkoxides. Various examples of such processes are known and can be used. For example, a procedure for forming an oxide film on metal substrate is disclosed in S.V. Lamaka et al., "TiOx self-assembled networks prepared by templating approach as nanostructured reservoirs for self-healing anticorrosion pre-treatments", Electrochemistry Communications, 8 (2006), pp. 421-428, which is incorporated herein by reference. A TiOₓ based sol is prepared at room temperature by hydrolysis of tetraisopropyl orthotitanate (Ti(OCH(CH₃)₂)₄ or Ti(OiPr)₄). Ti(OiPr)₄ is added to ethanol solution of Pluronic F127 in 1:30 weight ratio and stirred for 1 hour to form a precursor. The precursor is then hydrolyzed by addition of acidified water (pH 1) in 1:100 precursor-to-water molar ratio. Both the adluminal and abluminal sides of the stent body 26 is then coated with the resultant sol. In one example, coating is accomplished by dipping the stent body 26 in the sol for a period of time (for example, 3 minutes), and then withdrawing the stent body 26 from the sol at a controlled speed (for example, 18 cm/min). The assembly of the stent body 26 coated with a TiOₓ based sol is then heated (for example, at 250°C) to above supercritical temperature of the sol to dry the coating to form a layer of titanium oxide film, which substantially replicates the porous structure of the stent body and is believed to bear an epitaxial relationship with the stent body.

The materials and processing parameters can be varied to achieve the desired properties of the ceramic coatings. For example, combinations of hydrostatic pressure and solvent type can be chosen to achieve a desired solubility of the metal salts (e.g., Ti(OiPr)₄) in the sol and to vary the supercritical temperature of the sol to achieve desired drying temperatures for the coating. For example, in certain applications, as set forth in more detail below, drugs can be incorporated into the sol during the sol-gel process to impregnate the final ceramic coating with the drugs. High-temperature drying may not be desirable in such applications as it may drive off the drugs from the coating. By using appropriate solvents and/or pressure for dissolving the metal salts, the coating can be dried at desirably low temperatures, for example 200°C or lower. For example, various solvothermal sol-gel processes can be used for low-temperature synthesis of drug eluting ceramic coating layers in stents.

Further examples of sol-gel processing for coating substrates are disclosed in J.C. Yu et al, "Enhanced photocatalytic activity of mesoporous and ordinary TiO2 thin films by sulfuric acid treatment", Applied Catalysis B: Environmental 36 (2002) 31-43; and A. Chougnet et al., "Substrates do influence the ordering of mesoporous thin films", J. Mater. Chem., 15 (2005), 3340-3345. All references above are incorporated herein by reference.

The process outlined above is capable of producing ceramic coating on the order of a few micrometers thick or thicker. Further, the process can result in different surface morphologies for different surface portions of the same stent. In one example, the titanium oxide layer 32 on the smooth adluminal surface 28 of the stent body 26 has a uniform thickness with densely packed titanium oxide particles having a narrow size distribution. In contrast, the titanium oxide layer 36 on the etched abluminal surface 30 of the stent body 26 has a highly porous, network-like structure. The resultant relatively smooth adluminal surface 34 of the uniform layer 32 is conducive to endothelial cell grown thereon. In contrast, the porous abluminal surface layer 36 has a high surface area and is thus suitable for storing drugs.

### C. Materials Used

Various materials can be used to make stents with ceramic coating layers with portions of differing porosity. The stent body 26, for example, can be made of a variety of materials that are known, or later found, to be suitable for endoluminal implantation applications. For example, a variety of metals that have requisite mechanical properties (such as strength and deformability) are biocompatible and suitable as substrates for forming ceramic coatings can be used. Such metals include various alloys and other metals. In one configuration, the stent body 26 is made of stainless steel.

The ceramic layers, such as those labeled 32 and 36 can be made of a variety of ceramic materials that are biocompatible and compatible with the drug or drugs intended to be stored in the ceramic layers. Ceramic materials are solids that have as their essential component, and are composed in large part of, inorganic nonmetallic materials. Examples of suitable ceramic materials include certain transition metal oxides, such as titanium (TiOₓ), tantalum oxide (TaOₓ) and iridium oxide (IrOₓ). Iridium oxide can be particularly useful for intravascular stent applications because it exhibits good vascular compatibility.

As stated above, ceramic layers coating stent bodies can be formed to store and release drugs, i.e., therapeutic agents. Drugs can be loaded into the porous surface layer (e.g. ceramic layer 36) by a variety of suitable processes. In one example, one or more drugs are dissolved in water or an organic solvent, and a stent with ceramic coatings as described above is soaked in the solution for a period of time. The drug-loaded stent is subsequently dried off by evaporation, either at elevated temperatures or room temperature.

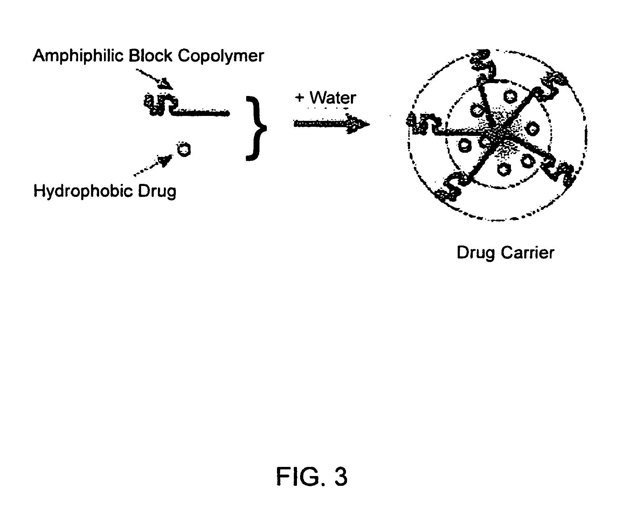

In another example, the drugs to be loaded into a stent are incorporated into the sol during the sol-gel process of making the ceramic coating layers. For example, drugs can be added to the ethanol solution of titanium alkoxide precursors, such as Ti(OiPr)₄ and polymers such as Pluronic or PEG during the sol-gel process. In one configuration, the drug molecules form complexes, such as micelles, with the components of the precursors, resulting in a more intimate association between the drug molecules and the ceramic layer, thereby forming a more robust drug eluting ceramic layer. For example, hydrophobic molecules of Paclitaxel, a chemotherapy drug, can form complexes with the amphiphilic block copolymers of Pluronic or PEG, as schematically illustrated in Figure 3. In such applications, it is often desirable to have a sol with a low supercritical temperature so that the drying process does not drive off the drug molecules, which are typically of low molecular weight. Low supercritical temperatures can be achieved by proper choice of solvothermal processes, as discussed above.

In a further configuration, hybrid surface coating layers 32, 36 can be formed, with both polymeric and ceramic materials present in the layers. Such layers can be made by, for example, dissolving ceramic precursor, as discussed above, as well as polymer in the sol. The resultant surface coating layers interpenetrating ceramic and polymeric networks. The presence of polymers in the surface layers provides at least two benefits in many applications. First, polymers can provide better bonding sites for drugs, which are typically organic, and compared to ceramic materials and thus a more robust drug reservoir. Second, the presence of a polymer network tends to make the surface coating layer more resilient, thereby enabling the surface coating layers to better accommodate the deformation of the stent during deployment.

It should be noted that, although the ceramic coating layers are disposed over abluminal or adluminal surfaces of stent bodies according to certain aspects of the present disclosure, steps of formation of ceramic coating layers need not be carried out while the stent body is in tubular form.

### III. Summary

Thus, ceramic coatings on stent bodies can be produced, with different surface morphologies coexisting in the same stent. Certain surface portions of the stent can be made relatively smooth to be more conducive to endothelial cell growth, thereby enhancing the incorporation of the stent into the host body tissue. Other surface portions of the stent can be made to be porous, for example, by etching the stent body portions upon which ceramic coatings are formed, to be more efficient in storing drugs. The sol-gel process for forming the ceramic coating results in strong bonding between the stent body and the ceramic coating, thereby enhancing the integrity of the coating and reducing the risk of breakage of the coating when the stent undergoes deformation during deployment.

The above specification, examples and data provide a complete description of the manufacture and use of the composition of the invention. Since many embodiments of the invention can be made without departing from the spirit and scope of the invention, the invention resides in the claims hereinafter appended.

## Claims

1. A method of making a stent (20), the method comprising:
- providing a tubular stent body (26) comprising an abluminal surface (30) and an adluminal surface (28);
- making at least a second surface portion comprising at least a portion of an abluminal surface (30) portion of the metallic stent rougher than at least a first surface portion comprising at least a portion of an adluminal surface (28) portion;
- coating the first surface portion of the metallic stent body material comprising coating the adluminal surface (28) with a first layer (32) comprising ceramic material; and
- coating the second surface portion of the metallic stent body material comprising coating the abluminal surface (30) portion with a second layer (36) comprising ceramic material, the second layer (36) being more porous than the first layer (32) by at least about 50%.

2. The method of claim 1, wherein making the second surface portions rougher than the first surface portions comprises etching the second surface portions.

3. The method of claim 2, wherein etching comprises chemical etching.

4. The method of claim 3, wherein chemical etching comprises masking the first surface portions with etching resist.

5. The method of claim 1, wherein coating the first and second surface portions with the first and second layers (32; 36), respectively, comprises coating the first and second surface portions with sol of ceramic precursor, and subsequently drying the ceramic precursor coated on the surface portions.

6. The method of claim 1, further comprising incorporating at least one drug into at least the second layer (36).

7. The method of claim 5, further comprising incorporating at least one drug into the sol of ceramic precursor.

8. The method of claim 1, wherein each of the first surface portion has a first surface structure, and the second surface portion has a second surface structure, coating the first and second surface portions comprising substantially replicating the first surface structure by coating the first surface portion with at least a sol of ceramic precursor, and subsequently drying the ceramic precursor and substantially replicating the second surface structure by coating the second surface portion with at least a sol of ceramic precursor, and subsequently drying the ceramic precursor.

9. A stent (20), comprising:
- a metallic stent body (26) comprising an abluminal surface (30) and an adluminal surface (28) and being deformable from a collapsed state to an expanded state and having at least a first surface portion and at least a second surface portion, the first surface portion being an etched portion and more porous than the second portion by at least 50%, wherein the second surface portion comprises at least a portion of an adluminal surface (28) portion of the stent body (26), and the at least one etched surface portion comprises at least a portion of an abluminal (30) surface portion of the stent body (26);
- a first layer (32) coating the first surface portion comprising the adluminal surface (28) portion, the first layer (32) comprising at least a ceramic material; and
- a second layer (36) coating the second surface portion comprising the abluminal (30) surface portion, the second layer (36) comprising at least a ceramic material.

10. The stent of claim 9, wherein the etched surface portion comprises a chemically etched surface portion or a laser etched surface portion.

11. The stent of claim 9, wherein first and second layers (32; 36) are made by sol-gel process.

12. The stent of claim 9, wherein the second layer further comprises one or more drugs incorporated therein.

13. The stent of claim 12, wherein the second layer is made by sol-gel process, and wherein the one or more drugs are incorporated into the second layer during the sol-gel process.

14. The stent of claim 9, wherein at least a portion of the first layer is epitaxial with the stent body, and wherein at least a portion of the second layer is epitaxial with the stent body.

15. The stent of claim 9, wherein each of the first and second layers comprises titanium oxide, tantalum oxide or iridium oxide.

16. The stent of claim 9, wherein each of the first and second layers (32; 36) further comprises polymer.

## Patentansprüche

1. Verfahren zum Herstellen eines Stents (20), wobei das Verfahren umfasst:
- Bereitstellen eines röhrenförmigen Stentkörpers (26), der eine abluminale Oberfläche (30) und eine adluminale Oberfläche (28) umfasst;
- Herstellen zumindest eines zweiten Oberflächenbereichs, der zumindest einen Bereich eines Bereichs der abluminalen Oberfläche (30) des metallischen Stents umfasst, der rauer ist, als zumindest ein erster Oberflächenbereich, der zumindest einen Bereich eines Bereichs der adluminalen Oberfläche (28) umfasst;
- Beschichten des ersten Oberflächenbereichs des metallischen Stentkörpermaterials, umfassend Beschichten der adluminalen Oberfläche (28) mit einer ersten Schicht (32), die ein keramisches Material umfasst; und
Beschichten des zweiten Oberflächenbereichs des metallischen Stentkörpermaterials, umfassend Beschichten des Bereichs der abluminalen Oberfläche (30) mit einer zweiten Schicht (36), die ein keramisches Material umfasst, wobei die zweite Schicht (36) um zumindest ungefähr 50% poröser ist als die erste Schicht (32).

2. Verfahren gemäß Anspruch 1, wobei rauer-Machen als die ersten Oberflächenbereiche der zweiten Oberflächenbereiche Ätzen der zweiten Oberflächenbereiche umfasst.

3. Verfahren gemäß Anspruch 2, wobei Ätzen chemisches Ätzen umfasst.

4. Verfahren gemäß Anspruch 3, wobei chemisches Ätzen Maskieren der ersten Oberflächenbereiche mit Ätzabdecklack umfasst.

5. Verfahren gemäß Anspruch 1, wobei Beschichten der ersten und zweiten Oberflächenbereiche mit den ersten und zweiten Schichten (32; 36), jeweils Beschichten der ersten und zweiten Oberflächenbereiche mit Sol eines keramischen Precursors umfasst, und darauffolgend Trocknen des keramischen Precursors, der auf die Oberflächenbereiche beschichtet ist.

6. Verfahren gemäß Anspruch 1, weiter umfassend Einlagern zumindest eines Arzneistoffs in zumindest die zweite Schicht (36).

7. Verfahren gemäß Anspruch 5, weiter umfassend Einlagern zumindest eines Arzneistoffs in das Sol des keramischen Precursors.

8. Verfahren gemäß Anspruch 1, wobei jeweils der erste Oberflächenbereich eine erste Oberflächenstruktur aufweist und der zweite Oberflächenbereich eine zweite Oberflächenstruktur aufweist, wobei Beschichten der ersten und zweiten Oberflächenbereiche im Wesentlichen Replizieren der ersten Oberflächenstruktur umfasst durch Beschichten des ersten Oberflächenbereichs mit zumindest einem Sol eines keramischen Precursors und darauffolgend Trocknen des keramischen Precursors und darauffolgend Replizieren der zweiten Oberflächenstruktur durch Beschichten des zweiten Oberflächenbereichs mit zumindest einem Sol eines keramischen Precursors und darauffolgend Trocknen des keramischen Precursors.

9. Ein Stent (20), umfassend:
- einen metallischen Stentkörper (26), der eine abluminale Oberfläche (30) und eine adluminale Oberfläche (28) umfasst und der verformbar ist von einem zusammengelegten Zustand zu einem expandierten Zustand und der zumindest einen ersten Oberflächenbereich und zumindest einen zweiten Oberflächenbereich aufweist, wobei der erste Oberflächenbereich ein geätzter Bereich ist und um zumindest 50% poröser ist als der zweite Bereich, wobei der zweite Oberflächenbereich zumindest einen Bereich eines adluminalen Oberflächen- (28) -bereichs des Stentkärpers (26) umfasst, und der zumindest eine geätzte Oberflächenbereich zumindest einen Bereich eines Bereichs der abluminalen Oberfläche (30) des Stentkörpers (26) umfasst;
- eine erste Schicht (32), die den ersten Oberflächenbereich beschichtet, die den Bereich der adluminalen Oberfläche (28) umfasst, wobei die erste Schicht (32) zumindest ein keramisches Material umfasst; und
- eine zweite Schicht (36), die den zweiten Oberflächenbereich beschichtet, der den Bereich der abluminalen Oberfläche (30) umfasst, wobei die zweite Schicht (36) zumindest ein keramisches Material umfasst.

10. Stent gemäß Anspruch 9, wobei der geätzte Oberflächenbereich einen chemisch geätzten Oberflächenbereich oder einen lasergeätzten Oberflächenbereich umfasst.

11. Stent gemäß Anspruch 9, wobei erste und zweite Schichten (32; 36) durch einen Sol-Gel-Prozess hergestellt werden.

12. Stent gemäß Anspruch 9, wobei die zweite Schicht weiter eine oder mehrere Arzneistoffe darin eingelagert umfasst.

13. Stent gemäß Anspruch 12, wobei die zweite Schicht durch einen Sol-Gel-Prozess hergestellt ist, und wobei die einen oder mehreren Arzneistoffe in die zweite Schicht während des Sol-Gel-Prozesses eingelagert werden.

14. Stent gemäß Anspruch 9, wobei zumindest ein Bereich der ersten Schicht epitaktisch mit dem Stentkörper ist, und wobei zumindest ein Bereich der zweiten Schicht epitaktisch mit dem Stentkörper ist.

15. Stent gemäß Anspruch 9, wobei jede der ersten und zweiten Schichten Titanoxid, Tantaloxid oder Iridiumoxid umfasst.

16. Stent gemäß Anspruch 9, wobei jede der ersten und zweiten Schichten (32, 36) weiter ein Polymer umfasst.

## Revendications

1. Procédé de fabrication d'un stent (20), le procédé consistant à :
- fournir un corps tubulaire de stent (26) comportant une surface abluminale (30) et une surface adluminale (28) ;
- fabriquer au moins une seconde portion de surface comportant au moins une portion d'une portion de surface abluminale (30) du stent métallique plus dure qu'au moins une première portion de surface comportant au moins une portion d'une portion de surface adluminale (28) ;
- revêtir la première portion de surface du matériau de corps de stent métallique comportant le revêtement de la surface adluminale (28) avec une première couche (32) comportant un matériau céramique ; et
- revêtir la seconde portion de surface du matériau de corps de stent métallique comportant le revêtement de la surface abluminale (30) avec une seconde couche (36) comportant un matériau céramique, la seconde couche (36) étant plus poreuse que la première couche (32) d'au moins environ 50%.

2. Procédé selon la revendication 1, dans lequel la fabrication des portions de seconde surface plus dures que les portions de première surface comporte l'attaque des portions de seconde surface.

3. Procédé selon la revendication 2, dans lequel l'attaque comprend l'attaque chimique.

4. Procédé selon la revendication 3, dans lequel l'attaque chimique comporte le masquage des portions de première surface avec un résistant à l'attaque.

5. Procédé selon la revendication 1, dans lequel le revêtement des portions de première et seconde surfaces avec les première et seconde couches (32 ; 36), respectivement, comporte le revêtement des portions de première et seconde surface avec un sel de précurseur de céramique, et par la suite le séchage du précurseur de céramique revêtu sur les portions de surface.

6. Procédé selon la revendication 1, comportant en outre l'incorporation d'au moins un médicament dans au moins la seconde couche (36).

7. Procédé selon la revendication 5, comportant en outre l'incorporation d'au moins un médicament dans le sel de précurseur de céramique.

8. Procédé selon la revendication 1, dans lequel chacune de la portion de première surface possède une structure de première surface, et de la portion de seconde surface possède une structure de seconde surface, revêtir les portions de première et seconde surface comportant essentiellement la reproduction de la structure de première surface par revêtement de la portion de première surface avec au moins un sel de précurseur de céramique, et par la suite à sécher le précurseur de céramique et essentiellement à reproduire la structure de seconde surface par revêtement de la portion de seconde surface avec au moins un sel de précurseur de céramique, et par la suite à sécher le précurseur de céramique.

9. Stent (20) comportant :
- un corps (26) de stent métallique comportant une surface abluminale (30) et une surface adluminale (28) et étant déformable à partir d'un état replié à un état expansé et ayant au moins une portion de première surface et au moins une portion de seconde surface, la portion de première surface étant une portion attaquée et d'au moins 50% plus poreuse que la seconde portion, dans lequel la portion de seconde surface comporte au moins une portion d'une portion de surface adluminale (28) du corps (26) de stent ;
- une première couche (32) revêtant la portion de première surface comportant la portion de surface adluminale (28), la première couche (32) comportant au moins un matériau céramique ; et
- une seconde couche (36) revêtant la portion de seconde surface comportant la portion de surface abluminale (30), la seconde couche (36) comportant au moins un matériau céramique.

10. Stent selon la revendication 9, dans lequel la portion de surface attaquée comporte une portion de surface attaquée chimiquement ou portion de surface attaquée au laser.

11. Stent selon la revendication 9, dans lequel les première et seconde couches (32 ; 36) sont fabriquées par un procédé sol-gel.

12. Stent selon la revendication 9, dans lequel la seconde couche comporte en outre un ou plusieurs médicaments incorporés à l'intérieur.

13. Stent selon la revendication 12, dans lequel la seconde couche est réalisée par un procédé sol-gel, et dans lequel un ou plusieurs médicaments sont incorporés dans la seconde couche lors du procédé sol-gel.

14. Stent selon la revendication 9, dans lequel au moins une portion de la première couche est épitaxiale avec le corps de stent, et dans lequel au moins une portion de la seconde couche est épitaxiale avec le corps de stent.

15. Stent selon la revendication 9, dans lequel chacune des première et second couches comporte de l'oxyde de titane, de l'oxyde de tantale ou de l'oxyde d'iridium.

16. Stent selon la revendication 9, dans lequel chacune des première et seconde couches (32 ; 36) comporte en outre du polymère.
